# EUROPEAN PATENT APPLICATION

(11) **EP 4 446 409 A1**
(43) Date of publication of application: **16.10.2024**
(21) Application number: 22904347.6
(22) Date of filing: 09.12.2022
(51) Int. Cl.: C12N 9/38

(54) **LACTASE SOLUTION**

(30) Priority: 09.12.2021 JP 2021199699
(71) Applicant: Godo Shusei Co., Ltd., Matsudo-shi, Chiba 271-0064 (JP)
(72) Inventor: AOKI, Shohei, Matsudo-shi, Chiba 271-0064 (JP); OGASAWARA, Junki, Matsudo-shi, Chiba 271-0064 (JP); SHINODA, Misaki, Matsudo-shi, Chiba 271-0064 (JP); BABA, Masahiro, Matsudo-shi, Chiba 271-0064 (JP)
(74) Representative: Hoffmann Eitle
(86) International application number: PCT/JP2022/045576
(87) International publication number: WO 2023/106420

(57) **Abstract**

An object of the present invention is to provide a lactase solution showing good permeation through a filtration filter, a lactase solution showing good permeation through a filtration filter and having a good residual lactase activity, and a lactase solution showing good permeation through a filtration filter and having a good residual lactase activity even when a lactase solution having a low protease activity and a low arylsulfatase activity as contaminant enzymes is used.

A lactase solution containing any of (i) to (iii) below.
(i) 0.0001 to 0.1 mass% of an unsaturated fatty acid or a salt thereof
(ii) 0.0001 to 0.1 mass% of a saturated fatty acid salt
(iii) 0.01 to 10 mass% of at least one selected from yeast extract, soybean peptone, pea protein, a casein degradation product, and corn steep liquor

## Description

### Technical Field

The present invention relates to a lactase solution which is improved with respect to clogging of a filtration filter.

### Background Art

Lactose intolerance refers to a condition in which various symptoms such as abdominal pain and diarrhea are exhibited when lactose is ingested. The cause is that lactose cannot be degraded well in the body. Lactose contained in milk or the like is degraded to galactose and glucose by lactase in the food manufacturing industry.

In order to degrade lactose contained in milk or the like, a lactase solution can be used. A lactase solution is commercialized by culturing a lactase-producing microorganism and removing intermixed substances derived from the culture, or the like.

When a protease acts on a protein or a peptide contained in milk, bitterness may occur or a flavor may be reduced in milk or dairy products. Therefore, it is desired that a lactase solution contains less proteases which are contaminant enzymes. Examples of a method for reducing proteases contained in a lactase solution include a method in which proteases are thermally inactivated (for example, PTL 1) and a method in which proteases are selectively adsorbed by an ion exchange resin (for example, PTL 2). Although there has been a demand for reduction of contaminant proteases contained in a lactase solution for a long time, with diversification of usage of a lactase solution in dairy product manufacturers and the like, the requirement level is higher than before. Arylsulfatase is known as a contaminant enzyme other than proteases contained in a lactase solution (for example, PTL 3). When arylsulfatase is added to milk, an off-flavor is likely to occur over time.

### Citation List

### Patent Literature

Patent Literature 1: JP S62-054471 B
Patent Literature 2: JP 6341911 B2
Patent Literature 3: JP 5544088 B2

### Summary of Invention

### Technical Problem

By improving the purification conditions, a lactase solution (highly purified product) having a low protease activity and a low arylsulfatase activity as contaminant enzymes could be obtained. When this was exported from Japan to foreign countries, it was found that the permeability when the lactase solution was allowed to pass through a filtration filter deteriorated. It was surprising that the permeability of the filtration filter to the highly purified lactase solution deteriorated. This is because, based on the disclosure of the invention in which the permeability of a filtration filter to a lactase solution is improved by setting the concentrations of polysaccharides and oligosaccharides contained in the lactase solution to a certain value or less (JP 2004-534527 A), it is expected that the more the lactase solution is purified, the more the permeability of the filtration filter is improved. The permeability of the filtration filter is required mainly when a lactase solution is added to milk after sterilization. If the permeability of a filtration filter is poor, the frequency of replacement of the filtration filter increases, the filter replacement cost increases and the production time is prolonged, which may affect the productivity of dairy products such as milk.

An object of the present invention is to provide a lactase solution showing good permeation through a filtration filter.

An object of the present invention is to provide a lactase solution showing good permeation through a filtration filter and having a good residual lactase activity.

An object of the present invention is to provide a lactase solution showing good permeation through a filtration filter and having a good residual lactase activity even when a lactase solution having a low protease activity and a low arylsulfatase activity as contaminant enzymes is used.

### Solution to Problem

The present invention has the following technical configuration, thereby solving the problem of the present invention.

(1) A lactase solution containing any of (i) to (iii) below.
   (i) 0.0001 to 0.1 mass% of an unsaturated fatty acid or a salt thereof
   (ii) 0.0001 to 0.1 mass% of a saturated fatty acid salt
   (iii) 0.01 to 10 mass% of at least one selected from yeast extract, soybean peptone, pea protein, a casein degradation product, and corn steep liquor
(2) The lactase solution according to (1), in which the unsaturated fatty acid (i) is at least one selected from oleic acid, vaccenic acid, linoleic acid, linolenic acid, eleostearic acid, mead acid, arachidonic acid, and nervonic acid.
(3) The lactase solution according to (1) or (2), in which the unsaturated fatty acid (i) has 18 carbon atoms.
(4) The lactase solution according to (1), in which a saturated fatty acid constituting the saturated fatty acid salt (ii) is a long-chain fatty acid having 12 to 18 carbon atoms.
(5) The lactase solution according to any one of (1) to (4), which has an arylsulfatase activity of 5 U/g or less.
(6) The lactase solution according to any one of (1) to (5), which has a protease activity of 5 U/g or less.
(7) The lactase solution according to any one of (1) to (6), containing a stabilizer.
(8) The lactase solution according to any one of (1) to (7), in which a slope (-a) after shaking is less than 0.5000.
(9) A packaged lactase solution, obtained by packaging the lactase solution according to any one of (1) to (8) in a container.

### Advantageous Effects of Invention

According to the present invention, it is possible to provide a lactase solution showing good permeation through a filtration filter.

According to the present invention, it is possible to provide a lactase solution showing good permeation through a filtration filter and having a good residual lactase activity.

According to the present invention, it is possible to provide a lactase solution showing good permeation through a filtration filter and having a good residual lactase activity even when a lactase solution having a low protease activity and a low arylsulfatase activity as contaminant enzymes is used.

### Description of Embodiments

The lactase solution of the present invention is characterized by containing any of (i) to (iii) below.
(i) 0.0001 to 0.1 mass% of an unsaturated fatty acid or a salt thereof
(ii) 0.0001 to 0.1 mass% of a saturated fatty acid salt
(iii) 0.01 to 10 mass% of at least one selected from yeast extract, soybean peptone, pea protein, and corn steep liquor

The incorporation of the above-mentioned components can provide a lactase solution showing good permeation through a filtration filter.

### <Unsaturated fatty acid>

When an unsaturated fatty acid is added to a lactase solution, the lactase solution becomes cloudy and a suspended substance is generated depending on the addition amount or type of the unsaturated fatty acid. In order to prevent this, it is preferred to add an unsaturated fatty acid to a lactase solution after the unsaturated fatty acid is dispersed in a dispersant.

An unsaturated fatty acid salt can be obtained by adding a base to an unsaturated fatty acid. As the base, for example, a hydroxide of an alkali metal salt can be used, and specifically, sodium hydroxide or potassium hydroxide can be used. As the unsaturated fatty acid salt, one excellent in dispersibility or solubility in a lactase solution is preferably used, and unsaturated fatty acid sodium or unsaturated fatty acid potassium is preferably used. The unsaturated fatty acid salt may be directly added to a lactase solution.

Both the unsaturated fatty acid and the unsaturated fatty acid salt improve the permeability of a filtration filter to a lactase solution. It is considered that the unsaturated fatty acid dispersed or dissolved in a lactase solution has some action. It is presumed that this unsaturated fatty acid masks a factor that deteriorates the permeability of a filtration filter. The unsaturated fatty acid is a so-called oil, and it was surprising that this improves the permeability of a filtration filter to a lactase solution.

Both the unsaturated fatty acid and the unsaturated fatty acid salt can provide a lactase solution having a good residual lactase activity.

The content of the unsaturated fatty acid or the unsaturated fatty acid salt contained in a lactase solution is preferably 0.0001 to 0.1 mass%, more preferably in the range of 0.0005 to 0.05 mass%, still more preferably in the range of 0.001 to 0.01 mass%, and particularly preferably in the range of 0.005 to 0.01 mass%.

By setting the upper limit of the content of the unsaturated fatty acid or the unsaturated fatty acid salt contained in a lactase solution to the above value, the permeability of a filtration filter to the lactase solution can be further enhanced.

As the unsaturated fatty acid, for example, oleic acid, vaccenic acid, linoleic acid, linolenic acid, eleostearic acid, mead acid, arachidonic acid, nervonic acid, or the like can be used. These may be used alone or two or more of these may be used in admixture. Among these, an unsaturated fatty acid having 18 carbon atoms is preferably used. It provides excellent permeability of a filtration filter and excellent storage stability of the lactase activity.

The number of unsaturated bonds constituting the unsaturated fatty acid is not particularly limited. A similar effect can be obtained regardless of the number of unsaturated bonds. The number of unsaturated bonds can be selected, for example, from 1 to 6, 1 to 3, or 1 to 2.

An unsaturated fatty acid and a saturated fatty acid described later may be used in admixture, or an oil derived from a natural product containing a plurality of these may be used. Examples of the oil derived from a natural product include soybean oil, corn oil, rice oil, rapeseed oil, and sunflower oil.

When an unsaturated fatty acid is directly added to a lactase solution, the lactase solution tends to be cloudy. When an unsaturated fatty acid is used, it is preferred to add an unsaturated fatty acid to a lactase solution after the unsaturated fatty acid is dispersed in a dispersant.

### (Dispersant)

As the dispersant for dispersing the unsaturated fatty acid, glycerin or ethanol can be used.

The addition amount of the dispersant is preferably 1 to 100,000 parts by mass with respect to 1 part by mass of the unsaturated fatty acid. The unsaturated fatty acid does not need to be dissolved in the dispersant, and may be in a cloudy state. It does not matter if cloud occurs in a lactase solution when the unsaturated fatty acid is dispersed in the dispersant and then mixed with the lactase solution.

When glycerin is used as the dispersant, the content is preferably in the range of 100 to 10,000 parts by mass, and more preferably in the range of 1,000 to 5,000 parts by mass with respect to 1 part by mass of the unsaturated fatty acid. When ethanol is used as the dispersant, the content is preferably in the range of 5 to 1,000 parts by mass, more preferably in the range of 10 to 500 parts by mass with respect to 1 part by mass of the unsaturated fatty acid.

### <Saturated fatty acid>

A saturated fatty acid may be solid at room temperature. Even when a solid saturated fatty acid is added to a lactase solution, it is hardly dissolved, and the permeability of a filtration filter cannot be improved. Rather, the permeability of a filtration filter deteriorates.

A saturated fatty acid salt can be obtained by adding a base to a saturated fatty acid to cause a reaction. As this base, one similar to those described for the unsaturated fatty acid can be used.

When a saturated fatty acid salt is added to a lactase solution, the permeability of the filtration filter can be improved. It is considered that the saturated fatty acid dispersed or dissolved in a lactase solution has some action. It is presumed that this saturated fatty acid masks a factor that deteriorates the permeability of a filtration filter. The saturated fatty acid is a so-called oil, and it was surprising that this improves the permeability of a filtration filter to a lactase solution.

The content of the unsaturated fatty acid salt contained in a lactase solution is preferably 0.0001 to 0.1 mass%, more preferably in the range of 0.0005 to 0.05 mass%, still more preferably in the range of 0.001 to 0.01 mass%, and particularly preferably in the range of 0.005 to 0.01 mass%.

By setting the content of the unsaturated fatty acid salt contained in a lactase solution to 0.1 mass% or less, the permeability of a filtration filter to the lactase solution can be further enhanced.

Examples of the saturated fatty acid include butyric acid, valeric acid, caproic acid, enanthic acid, caprylic acid, pelargonic acid, capric acid, lauric acid, myristic acid, pentadecylic acid, palmitic acid, margaric acid, stearic acid, arachidic acid, behenic acid, lignoceric acid, cerotic acid, montanic acid, and melissic acid. These may be used alone or two or more of these may be used in admixture. The number of carbon atoms in the saturated fatty acid is preferably in the range of 8 to 20, more preferably in the range of 10 to 16, and still more preferably in the range of 12 to 14. Although it depends on the composition of the lactase solution, when a saturated fatty acid is used, the permeability of a filtration filter can be further enhanced by setting the number of carbon atoms in the saturated fatty acid to 8 or more. Meanwhile, by setting the number of carbon atoms in the saturated fatty acid to 20 or less, the solubility of the saturated fatty acid can be sufficiently ensured, and the permeability of a filtration filter can be further enhanced.

### <Yeast extract and the like>

Commercially available yeast extract, soybean peptone, pea protein, casein degradation product, or corn steep liquor may be used as it is, or may be subjected to some treatment to increase the proportion of a required material.

Even when an amino acid alone or a tripeptide was added to a lactase solution, the effect of enhancing the permeability of a filtration filter to the lactase solution was not observed, and thus it is presumed that a molecular weight reduction treatment is not a preferred method. It is preferred to obtain a peptide or a protein having a large molecular weight by molecular weight fractionation or the like.

The amount of yeast extract, soybean peptone, pea protein, a casein degradation product, or corn steep liquor added to a lactase solution is preferably 0.01 to 10 mass%. By setting the lower limit of the addition amount to the above value, the permeability of a filtration filter to a lactase solution can be further enhanced. Meanwhile, by setting the upper limit of the addition amount to the above value, the cost can be reduced.

### <Lactase solution>

The lactase solution in the present invention may contain a protease equivalent to a conventional one, but it is preferred that the protease activity is lower than a conventional one. Since a plurality of proteases are often produced during culture, it is preferred to set the protease activity value contained in the lactase solution as a target. The protease activity of the lactase solution in the present invention is preferably 20 U/g or less, more preferably 5 U/g or less, and still more preferably 3 U/g or less or 1 U/g or less. It is particularly preferred that the protease activity is less than the lower detection limit value in the measurement method described later.

The protease activity means a value measured by a casein degradation method (Casein hydrolysis-UV method).

The lactase solution in the present invention may contain arylsulfatase equivalent to a conventional one, but it is preferred that the arylsulfatase activity is lower than a conventional one. The arylsulfatase activity of the lactase solution in the present invention is preferably 50 U/g or less, more preferably 10 U/g or less, and still more preferably 5 U/g or less or 3 U/g or less. It is particularly preferred that the arylsulfatase activity is less than the lower detection limit value (about 3 U/g) in the measurement method described later.

### (Lactase)

Lactase has been isolated from a very wide range of organisms including microorganisms. Lactase is often an intracellular or extracellular component of microorganisms such as Kluyveromyces and Bacillus. Kluyveromyces, in particular K. fragilis and K. lactis, and yeast in the genus Candida, the genus Torula, and the genus Torulopsis, and the like are common sources of yeast enzyme lactase, while B. coagulans or B. circulans is a well-known source of bacterial lactase. Several lactase preparations derived from these organisms are commercially available. These lactases are all so-called neutral lactases because the optimum pH is pH = 6 to pH = 8. Further, Aspergillus niger, Aspergillus oryzae, and Penicillium multicolor produce extracellular lactase, and US 5,736,374 describes examples of such lactase produced by Aspergillus oryzae. The enzymatic properties of lactase, such as optimal pH and optimal temperature, vary depending on species. In general, extracellular lactase is so-called acid lactase having an optimum pH as low as pH = 3.5 to pH = 5.0.

In addition, there is also lactase derived from Bifidobacterium bifidum that acts in a neutral and acidic (pH 4 to pH 10) environment.

It is also possible to produce lactase by recombining a lactase gene derived from such a microorganism into a host. Examples of the host include microorganisms in the genus Aspergillus, the genus Kluyveromyces, the genus Trichoderma, the genus Escherichia coli, the genus Pichia, the genus Saccharomyces, the genus Yarrowia, the genus Neurospora, the genus Lactococcus, and the genus Bacillus.

In the present invention, it is preferred to use neutral lactase and acid lactase, and it is particularly preferred to use neutral lactase derived from the genus Kluyveromyces, acid lactase derived from the genus Aspergillus, or lactase derived from Bifidobacteirum.

The lactase solution of the present invention may contain various components as needed. Specific examples thereof include metal salts, various saccharides, ascorbic acid, and glycerin that contribute to stabilization of lactase, starch and dextrin that are excipients for improving usability, and inorganic salts having a buffering action.

The activity of the lactase solution is preferably in the range of 100 to 1,000,000 U/g, more preferably in the range of 1,000 to 500,000 U/g, and still more preferably in the range of 10,000 to 110,000 U/g as measured by the measurement method described later. It is preferred that the activity is within this range before and after a packaged lactase solution is transported. As the activity of the lactase solution is lower, the permeability of a filtration filter tends to be worse after a packaged lactase solution is transported.

The lactase solution is preferably substantially transparent. This is because when cloud has occurred in the lactase solution, the lactase solution easily clogs a filtration filter. Substantially transparent means that it is only necessary that the lactase solution is not cloudy when it is visually inspected. The lactase solution may be colored. Specifically, the solution may be a pale yellow to pale brown solution.

The packaged lactase solution can be obtained by packaging the lactase solution in a container.

The temperature of the packaged lactase solution is preferably higher than 0°C and 20°C or lower. As the temperature during storage and transportation increases, aggregates are more likely to be generated in the lactase solution.

Immediately after the production of the lactase solution, protein aggregates are not contained in the lactase solution, but protein aggregates increase with an increase in storage period or through transportation. The protein aggregates are aggregates of lactase protein molecules, aggregates of a lactase protein molecule and other protein molecule, and aggregates of other protein molecules.

### (Stabilizer)

The amount of the stabilizer to be incorporated in the lactase solution is preferably 10 mass% to 90 mass%, more preferably 20 mass% to 80 mass%, further preferably 30 mass% to 70 mass%, and particularly preferably 40 mass% to 60 mass%. When the amount of the stabilizer is equal to or more than the lower limit value, it is easy to maintain the lactase activity of the lactase solution for a long period of time. When the amount of the stabilizer is more than the upper limit value, the viscosity of the lactase solution increases, and therefore the filtration time becomes long and the workability deteriorates.

Examples of the stabilizer include glycerin and sorbitol.

### «Method for producing lactase solution»

A method for producing a lactase solution includes, for example: (1) a step of extracting lactase involving destruction of cell walls after a microorganism such as yeast is cultured, and (2) a purification step for removing intermixed substances and the like derived from the culture from the extracted lactase. The method may include (3) a step of adding an additive to the lactase (which may be one prepared immediately before or a commercially available product) as needed, and (4) a filtering step for sterilization.

A packaged lactase solution can be obtained by filling a given container with a predetermined amount of the lactase solution after filtration.

### «Usage and application of lactase solution»

### (Usage of lactase solution)

As a specific utilization form of the lactase solution, for example, the lactase solution is used in the production of fermented milk. Examples of a method for producing lactose-degraded fermented milk include: 1. a method in which lactase is added to milk before sterilization to degrade lactose, and then lactase is inactivated simultaneously with heat sterilization of the milk, and then the milk is fermented (JP H05-501197 A); 2. a method in which lactase is added to sterilized milk to degrade lactose, and then lactase is inactivated by a heat treatment, and then the milk is fermented; 3. a method in which lactose in milk is degraded with immobilized lactase, and then the milk is fermented (JP S46-105593 A and JP S59-162833 A); and 4. a method in which a raw material obtained by previously degrading lactose or removing lactose is used for sterilized milk to carry out fermentation.

Further, as a specific utilization form of the lactase solution of the present invention, the lactase solution is used in the production of long life milk. The long life milk is long-term storage milk, and a production step includes a sterilization step and a continuous aseptic packaging step, and milk is generally treated by an ultra-high temperature short time sterilization method at 135 to 150°C for several seconds, and filled in a step in which a paper container sterilized in advance with hydrogen peroxide can be aseptically packaged.

The lactase solution to be added to long life milk is generally added after filtration sterilization when milk after ultra-high temperature and short time sterilization is filled.

### (Application of lactase solution)

The lactase solution according to the present invention is particularly suitable for production of a dairy product. Here, the dairy product refers to ice cream, milk such as long life milk, yoghurt, fresh cream, sour cream, cheese, and the like. In particular, the lactase solution according to the present invention is suitable for production of long life milk.

Hereinafter, the present invention will be described using examples, but the present invention is not limited thereto.

### Examples

In the following examples and the like, commercially available GODO-YNL2LS (manufactured by GODO SHUSEI CO., LTD.) was used as the lactase solution. GODO-YNL2LS was neutral lactase derived from Kluyveromyces, and had a lactase activity of 60,000 U/g, an aryl sulfatase activity of less than the lower detection limit value as measured by the following measurement method, a protease activity of less than the lower detection limit value as measured by the following measurement method, and a specific gravity of 1.18 (g/mL), and contained 50% (v/v) of glycerin.

### (Filter permeability test)

The detailed conditions in this example of the filter permeability test will be described in detail below.

### (Filter permeability measurement procedure)

The following operation was performed in an environment of 5 to 15°C.
1. A measuring apparatus, a lactase solution sample, and distilled water were cooled to the test environment temperature.
2. The lactase solution sample was diluted with distilled water or concentrated by ultrafiltration to adjust the lactase activity to 16,000 to 17,500 U/g and mixed well.
3. In the test, one in which a 25 mm stainless steel filter holder (manufactured by PALL Corporation, product number 1209 (effective membrane area: 3.7 cm²)) is connected to a stainless steel holder with a 47 mm tank (Advantec Toyo Kaisha, Ltd., product name "KST-47") was used as an apparatus. A sample permeating portion in the filter holder is configured to include an O-ring, a membrane, a support screen, and an underdrain disc from the measurement sample inlet side (in the test of the present invention, the filter of the stainless steel holder with the tank and the parts of the filter holding portion (the support screen and the support thereof) were not attached). As the membrane, DURAPORE (product name) (pore diameter: 0.22 µm, ϕ25 mm, made of hydrophilic PVDF) manufactured by Merck Millipore was used, and as the support screen, Type 316 stainless steel attached to the filter holder was used. In addition, in order to adjust the permeation rate, four circular label stickers (manufactured by A-One Co., Ltd., product name A-one color label 07010) having a diameter of 0.9 cm were stuck symmetrically to an upper portion of the support screen (measurement sample inlet side) (effective membrane area: 1.26 cm²), and a membrane filter (pore diameter: 0.22 µm) was set. The membrane was rinsed with a 50% glycerin aqueous solution and attached to the stainless steel filter holder.
4. The lactase solution sample (sample) diluted in 2. was charged into the stainless steel holder with the tank.
5. A pressure of 0.2 MPa was applied to the stainless steel holder with the tank using an air compressor (manufactured by Yaezaki Kuatsu Co., Ltd., product name "KAPSEL-CON YC-3R" or "PC4-15HLM"), and the enzyme solution was pumped. The permeate was received in a container such as a beaker, the amount of the permeate was recorded every 10 seconds, and (1) the permeation amount (permeate (kg/m²)) and (2) the permeation rate (flux (kg/min × m²)) per square meter of the membrane were determined by the following method. In addition, the slope a of an approximate curve y = ax + b obtained when (1) is plotted on the x axis and (2) is plotted on the y axis was determined.

### Calculation formula (concept formula)

Permeate (kg/m2) = weight (g) of product that permeated at point n/(membrane radius (mm)) × membrane radius (mm) × circumference ratio) (m2) × 1000 Flux (kg/min × m2) = (permeate at point n - permeate at point (n-1))/(permeation time (min) at point n - permeation time (min) at point (n-1)) *n indicates a measurement point. In order to record the permeation amount for every 10 seconds, for example, when the point n is for the permeation amount or permeate at the time of permeation for 10 seconds, the point (n-1) is for the permeation amount or permeate at the time of permeation for 0 seconds.

### (Residual activity of lactase activity)

The lactase activity of the below-mentioned lactase solution immediately after preparation and after storage at 50°C for 3 days was measured by the following method. The lactase activity immediately after preparation was taken as 100%, and the lactase activity after storage at 50°C for 3 days was expressed as a relative value (%).

### (Method for measuring lactase activity)

A diluted enzyme solution (0.5 mL) and 0.5 mL of a basic buffer (100 mM phosphate buffer (pH 6.5) containing 0.1 mM manganese chloride) are mixed, and the mixture is preheated at 37°C for 3 minutes, and then, 1.0 mL of a 1.65 mM O-nitrophenyl-β-D-galactopyranoside (ONPG) aqueous solution is added thereto to cause a reaction at 37°C for 1 minute. After 2.0 mL of a reaction stop solution (200 mM sodium carbonate) was added thereto, the absorbance at OD 420 nm was measured. One unit (U) is the enzyme activity that increases the OD 420 nm by 1 from the substrate in 10 minutes.

### (Measurement of arylsulfatase activity)

### (Measurement of arylsulfatase activity by colorimetric method)

To 0.5 mL of the lactase solution, 0.5 mL of a 100 mM potassium phosphate buffer (pH 6.5) solution of 20 mM p-nitrophenylsulfate was added to cause a reaction at 37°C for 3 hours. Then, 1.5 mL of a 1.5 N aqueous sodium hydroxide solution was added thereto to stop the reaction, and the absorbance at 410 nm was measured.

Separately, aqueous solutions containing p-nitrophenol at a concentration of 0 to 0.5 mM were prepared. To 0.5 mL of each of these solutions, 0.5 mL of a 100 mM potassium phosphate buffer (pH 6.5) solution was added, and 1.5 mL of a 1.5 N aqueous sodium hydroxide solution was further added thereto to obtain measurement samples. The absorbance at 410 nm was measured, and a calibration curve was prepared.

From the calibration curve, the concentration of p-nitrophenol contained in 1 mL of the reaction solution was determined and further divided by 3 (since the reaction time was 3 hours) to calculate the concentration of p-nitrophenol when the reaction time was 1 hour. Subsequently, from this concentration, the amount of p-nitrophenol contained in 1 mL of the reaction solution was calculated (unit: nmole), and the arylsulfatase activity was calculated by further doubling the calculated amount (in order to convert it per gram of the lactase preparation because the amount of the lactase preparation used was 0.5 g). One unit (1 U) is the activity that produces 1 nmole of p-nitrophenol per hour, and the arylsulfatase activity is represented in the unit "U/g-lactase solution".

### (Casein degradation method)

To 5 mL of a 0.6% casein aqueous solution (pH 6.5, a 50 mM potassium dihydrogen phosphate buffer containing 0.05 mM manganese chloride), 1 mL of a diluted enzyme solution is added to cause a reaction at 30°C for 10 minutes, and thereafter 5 mL of a trichloroacetic acid reagent (pH 4.0, 1.8% anhydrous sodium acetate, 1.8% trichloroacetic acid, 1.98% acetic acid) is added to stop the reaction, and the mixture is further allowed to stand at 30°C for 30 minutes, and filtered, and then the absorbance at 275 nm is measured. The amount of enzyme (enzyme activity) that releases an amino acid corresponding to 1 µg of tyrosine per minute under the conditions is defined as 1 protease unit (PU).

A predetermined amount of a given additive was added to GODO-YNL2LS, and the slope (-a) showing filter permeability and the residual activity of lactase activity were measured. The types of additives, the addition amount, the slope, and the residual activity are shown in tables. Table 1 shows the results of using a fatty acid or a fatty acid salt as an additive, and Table 2 shows the results of using a protein, a peptide, or an amino acid.

Each lactase solution was obtained by adding the additive shown in the table in a predetermined amount to 60,000 U/g GODO-YNL2LS. Each lactase solution immediately after preparation was subjected to a filter permeability test and a lactase activity measurement by the above-mentioned methods. The lactase activity of each lactase solution after storage at 50°C for 3 days was measured.

In 1000 mL I-Boy (wide mouth (manufactured by AS ONE Corporation, product number: 5-002-02)), 60 g of each lactase solution was placed and then shaken at 20°C with an amplitude of 30 mm and 100 spm for 1 hour. The lactase solution after shaking was allowed to stand for 1 hour, and subjected to a filter permeability test by the above-mentioned method.

The saturated fatty acids shown in the table are solid at room temperature and do not dissolve even when added to the lactase solution. A saturated fatty acid was converted into a saturated fatty acid salt by the following method (saponification), and then added to the lactase solution.

In a container, 0.03 g of a saturated fatty acid and 1 mL of a 1 M aqueous sodium hydroxide solution were mixed, and 4 mL of distilled water was added thereto. The mixture was subjected to a saponification treatment in boiling water at 100°C for 5 minutes, then left to cool to room temperature, and the reaction product was added to 294 g of a total amount of the lactase solution (GODO-YNL2LS) (addition amount: 0.01%). The filtrate obtained by filtering this was subjected to a filter permeability test and a residual activity of lactase activity.

When the unsaturated fatty acid shown in the table is directly added to the lactase solution, cloud occurs in the lactase solution. The unsaturated fatty acid was dispersed in glycerin as a dispersant and then added to the lactase solution as described below.

In this example, 108 g of 99% glycerin was mixed with 0.03 g of the unsaturated fatty acid in a container. The mixture was added to 192 g of a total amount of the lactase solution (GODO-YNL2LS) (addition amount: 0.01%). The filtrate obtained by filtering this was subjected to a filter permeability test and a residual activity of lactase activity.

The addition amount (%) of the additive shown in Table 1 was adjusted by changing the amount of the unsaturated fatty acid.

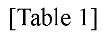

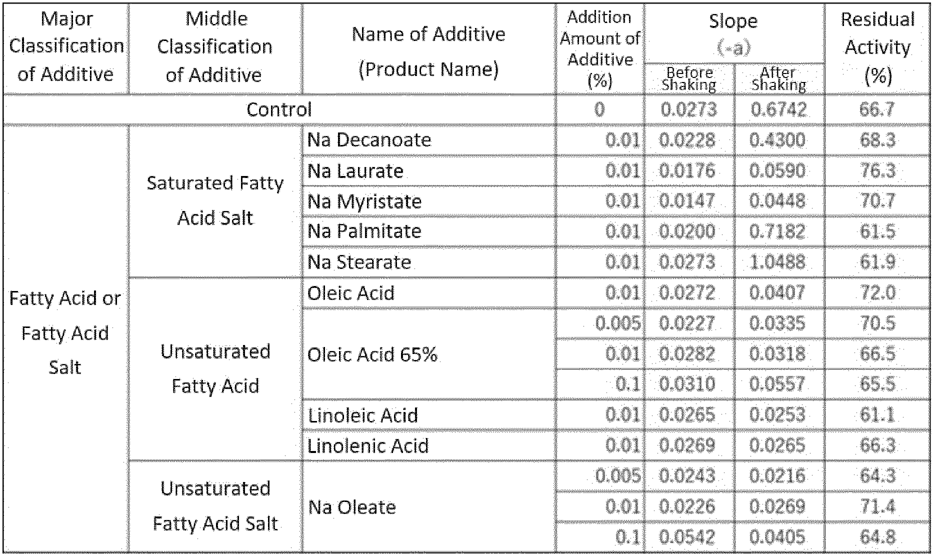

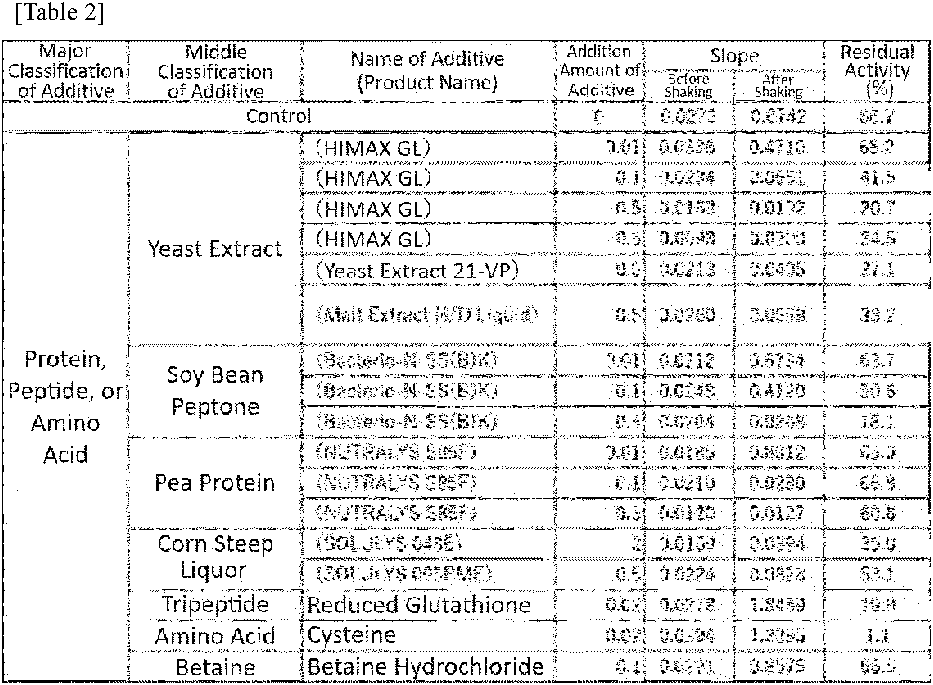

The slope (-a) after shaking is preferably less than 0.5000, more preferably less than 0.1000, still more preferably less than 0.0500, even more preferably less than 0.0300, and particularly preferably less than 0.0100 in consideration of actual product trade.

The residual activity of lactase activity is preferably 60% or more, more preferably 70% or more, still more preferably 80% or more, and particularly preferably 90% or more in consideration of actual product trade. The residual activity of lactase activity is preferably as close as possible to 100%.

Among the saturated fatty acid salts, in the case of Na palmitate and Na stearate, the slope after shaking deteriorated. This is because the saponification treatment was insufficient, and it was expected that the slope after shaking is improved by increasing the concentration of sodium hydroxide during the saponification treatment.

Based on the slope after shaking and the value of the residual activity of lactase activity, Na laurate, Na myristate, oleic acid, linoleic acid, linolenic acid, sodium oleate, and pea protein were promising as additives.

Oleic acid, linoleic acid, and linolenic acid, which were particularly promising, were subjected to the following storage stability test. Oleic acid, linoleic acid, and linolenic acid were added to a 60,000 U/g lactase solution so that the concentration was 0.005% and 0.01%, and the resulting mixtures were stored at 12°C for 30 days, and then the filter permeability test and measurement of the residual activity of lactase activity were performed. As a result, it was verified that both were at a practical level.

## Claims

1. A lactase solution comprising any of (i) to (iii) below:
(i) 0.0001 to 0.1 mass% of an unsaturated fatty acid or a salt thereof;
(ii) 0.0001 to 0.1 mass% of a saturated fatty acid salt; and
(iii) 0.01 to 10 mass% of at least one selected from yeast extract, soybean peptone, pea protein, a casein degradation product, and corn steep liquor.

2. The lactase solution according to claim 1, wherein the unsaturated fatty acid (i) is at least one selected from oleic acid, vaccenic acid, linoleic acid, linolenic acid, eleostearic acid, mead acid, arachidonic acid, and nervonic acid.

3. The lactase solution according to claim 1 or 2, wherein the unsaturated fatty acid (i) has 18 carbon atoms.

4. The lactase solution according to claim 1, wherein a saturated fatty acid constituting the saturated fatty acid salt (ii) is a long-chain fatty acid having 12 to 18 carbon atoms.

5. The lactase solution according to any one of claims 1 to 4, which has an arylsulfatase activity of 5 U/g or less.

6. The lactase solution according to any one of claims 1 to 5, which has a protease activity of 5 U/g or less.

7. The lactase solution according to any one of claims 1 to 6, comprising a stabilizer.

8. The lactase solution according to any one of claims 1 to 7, wherein a slope (-a) after shaking is less than 0.5000.

9. A packaged lactase solution, obtained by packaging the lactase solution according to any one of claims 1 to 8 in a container.
